# EUROPEAN PATENT SPECIFICATION

(11) **EP 3 127 570 B1**
(45) Date of publication and mention of the grant of the patent: **06.05.2020**
(21) Application number: 15773742.0
(22) Date of filing: 03.03.2015
(51) Int. Cl.: A61M 5/31, A61M 5/28, A61M 5/32, A61M 5/46

(54) **SYRINGE**
SPRITZE
SERINGUE

(30) Priority: 31.03.2014 JP 2014074959
(43) Date of publication of application: 08.02.2017
(73) Proprietor: Terumo Kabushiki Kaisha, Tokyo 151-0072 (JP)
(72) Inventor: OGAWA, Junichi, Nakakoma-gun Yamanashi 409-3853 (JP)
(74) Representative: Casalonga
(86) International application number: PCT/JP2015/056187
(87) International publication number: WO 2015/151692

(56) References cited:
- WO-A1-2013/146000
- WO-A1-2013/146000
- WO-A1-2014/045405
- JP-A- 2002 502 296
- JP-A- 2002 502 296
- JP-A- 2005 520 602
- JP-A- 2007 159 717
- JP-A- 2007 159 717
- JP-A- 2010 529 892
- JP-A- 2011 528 247
- US-A1- 2013 053 788

## Description

### Technical Field

The present invention relates to a syringe capable of stably administrating an injection liquid-drug into a subject person, such as a patient, and preventing slipping of a finger even when a plunger is pushed with a high pushing force.

### Background Art

A widely known type of syringe includes an syringe barrel flange protruding in opposite directions from a rim of a proximal opening of an syringe barrel and a disk-like pressing portion located on the proximal end of a plunger of which distal portion is inserted in the syringe barrel. A syringe as defined in the preamble of claim 1 is disclosed in JP2007159717. To administrate a liquid-drug into a subject person such as a patient, a user such as a doctor holds the syringe by placing the index finger and the middle finger on the syringe barrel flange and the thumb on the pressing portion. By pushing the pressing portion with the thumb, the liquid-drug is discharged from the syringe barrel through an injection needle piercing the skin of a patient to be administrated into the patient.

In administration of a high viscosity liquid-drug having low fluidity or intradermal administration, which requires a high pressure to inject a liquid-drug through a thin injection needle, the plunger needs to be pushed with a high pushing force. In such administration, the aforementioned syringe, which includes the injection needle and the syringe barrel flange provided as separate parts, cannot be held in a stationary manner by a user during administration of the liquid-drug.

Patent Literature 1 discloses a syringe that can be kept stable during administration of a liquid-drug even when a high plunger pushing force is required. The syringe includes a syringe barrel inserted in an outer tube and a pressing portion protruding from a proximal opening of the outer tube. A user holds the outer tube with four fingers which are the index finger, the middle finger, the fourth finger, and the fifth finger and pushes the pressing portion with the thumb to administrate a liquid-drug into a subject person.

### Citation List

### Patent Literature

Patent Literature 1: JP 1486702 S

### Summary of Invention

### Technical Problem

The syringe disclosed in Patent Literature 1 is such that, a high plunger pushing force causes the finger holding an outer tube to slip toward the proximal end of the outer tube, approaching the thumb. When this happens during administration of a liquid-drug, the index finger may be caught between the proximal opening of the outer tube and the pressing portion or the thumb may touch the index finger to hinder the push given to the pressing portion, which prevents the plunger to be fully pushed in. This results in failure of administrating all the specified dose of the liquid-drug stored in the syringe barrel.

The syringe barrel flange located on the outer tube to protrude in two directions may mistakenly be held with the index finger and the middle finger. If a user holds the syringe barrel flange in such a manner, a high plunger pushing force destabilizes the syringe during administration of the liquid-drug.

An object of the present invention is to provide a syringe that can firmly be held in a stable manner to administrate a liquid-drug into a subject person even when a high plunger pushing force is required and can be recognized by a user that the syringe is to be held in a grabbing manner when using.

### Solution to Problem

The present invention is made to achieve the aforementioned object and provides a syringe comprising: a syringe barrel including: a liquid-drug discharge portion on a distal end of the barrel, an opening on a proximal end of the barrel, and a body portion for storing a liquid-drug; a gasket inserted in the syringe barrel; and a plunger including: a shaft inserted in the syringe barrel from the opening and: a pressing portion located on a proximal end of the shaft, the pressing portion being pushable by a thumb to slide the gasket toward the liquid-drug discharge portion, wherein the syringe barrel further includes: a brim-shaped finger hooking portion protruding from an outer circumferential surface of the syringe barrel so as to extend in a first direction, which is a radial direction approximately perpendicular to a central axis of the syringe barrel, the finger hooking portion being configured to be hooked with a finger; and a sleeve-shaped gripping portion that is located between a distal end of the finger hooking portion and a proximal end of the liquid-drug discharge portion and is configured to be held with at least a middle finger, a fourth finger, and a fifth finger.

The syringe is preferably configured that the gripping portion has an overall length of at least 70 mm along the central axis and an outer circumferential length of 40 to 95 mm at least at a proximal portion of the gripping portion.

The syringe may be configured that the syringe barrel includes an annular protruding portion between the gripping portion and the opening, the annular protruding portion protruding outward from an outer circumferential surface of the gripping portion by 0.5 to 2 mm in a direction approximately perpendicular to the central axis, and the finger hooking portion extends the first direction so as to connect an outer circumferential surface of the annular protruding portion, the finger hooking portion protruding from the outer circumferential surface of the gripping portion by at least 4 mm.

The syringe is preferably configured that a width of the finger hooking portion along a second direction perpendicular to the first direction in the radial direction is equal to or larger than a width of the syringe barrel along the second direction.

The syringe may be configured that the syringe barrel further includes: an syringe barrel body including the liquid-drug discharge portion at a distal end of syringe barrel body, an insertion opening opened at a proximal end of syringe barrel body, and the body portion that is located between the liquid-drug discharge portion and the insertion opening and has a form of a tube with a maximum outer diameter of 12 mm and a maximum overall length of 60 mm along the central axis; and an outer tube extending at least from a periphery of the insertion opening to the opening so as to form a sleeve, wherein the outer tube includes the finger hooking portion, and the finger hooking portion is located at least 70 mm from a proximal end of the liquid-drug discharge portion and protrudes from an outer circumferential surface of the outer tube in the first direction, and wherein the outer tube has an outer circumferential length of 40 to 95 mm at least near a distal end of the finger hooking portion, and wherein the outer tube constitutes at least a proximal portion of the gripping portion.

The syringe is preferably used for intradermal administration of the liquid-drug.

A prefilled syringe may include: any one of the syringes; a cap detachably attached to the syringe barrel to seal the liquid-drug discharge portion; and a liquid-drug that is stored between the liquid-drug discharge portion and the gasket and is dischargeable through the liquid-drug discharge portion by sliding of the gasket.

The prefilled syringe is preferably configured that the syringe barrel includes: an syringe barrel body including the liquid-drug discharge portion at a distal end of syringe barrel body, an insertion opening opened at a proximal end of syringe barrel body, the body portion, and an syringe barrel flange that protrudes from a rim of the insertion opening, and wherein the body portion is located between the liquid-drug discharge portion and the insertion opening and has a form of a tube with a maximum outer diameter of 12 mm and a maximum overall length along the central axis of 60 mm; and an outer tube including a tube distal portion opened at a distal end of the outer tube and the opening at a proximal end of the outer tube, wherein the outer tube is formed of a sleeve in which the syringe barrel body is configured to be inserted, wherein the outer tube includes the finger hooking portion that is located at least 70 mm from a proximal end of the liquid-drug discharge portion and protrudes from an outer circumferential surface of the outer tube in the first direction, wherein the outer tube has an outer circumferential length of 40 to 95 mm at least near a distal end of the finger hooking portion, and wherein the outer tube unmovably holds the syringe barrel flange to support the syringe barrel body inside the outer tube, and the outer tube constitutes at least a proximal portion of the gripping portion.

The prefilled syringe may be configured that the outer tube extends from the rim of the insertion opening toward the distal end of the syringe barrel body so as to form a sleeve surrounding a side surface of the body portion, the outer circumferential length of the outer tube having a range of 40 to 95 mm.

The prefilled syringe is preferably configured that the outer tube includes an inspection hole penetrating a wall of the outer tube in a second direction perpendicular to the first direction in the radial direction, , the body portion being visually exposed through the inspection hole.

The prefilled syringe is preferably configured that the outer tube includes two pairs of ribs at locations opposing each other with the central axis therebetween, the ribs in each pair protruding from one side of an inner wall of the outer tube and linearly along the central axis, each of the two pair of ribs includes a contact portion and a support portion, the contact portion configured to contact with an outer circumferential surface of a distal portion of the syringe barrel body to prevent the syringe barrel body from tilting toward the outer tube, the support portion supporting a distal surface of the syringe barrel flange, and a distance between the two pairs of ribs opposing each other is greater than a width of the inspection hole along the first direction.

The prefilled syringe may be configured that the syringe barrel flange has an outer profile having a long axis and a short axis perpendicular to the long axis when viewed from a proximal side of syringe barrel flange, the syringe barrel flange including a pair of long-axis side surfaces located at both ends in the long axis direction and a pair of short-axis side surfaces located at both ends in the short axis direction, and the outer tube includes rotation-restricting ribs each protruding from the inner wall of the outer tube toward the short-axis side surface of the syringe barrel flange, each rotation-restricting rib being configured to contact a respective the short-axis side surface to restrict the syringe barrel body from rotating relative to the outer tube.

The prefilled syringe is preferably configured that the syringe barrel body includes a luer-lock adaptor that surrounds a side surface of the liquid-drug discharge portion and is configured to be screwably attached to a hub with an injection needle for administrating the liquid-drug by screwing.

The prefilled syringe may be configured that the outer tube includes the annular protruding portion located between the gripping portion and the opening, the annular protruding portion protruding outward from an outer circumferential surface of the outer tube by 0.5 to 2 mm, the finger hooking portion extends in the first direction so as to connect to an outer circumferential surface of the annular protruding portion, a side surface of the finger hooking portion includes two parallel linear sections extending along the first direction and an arc section connecting the two linear sections by extending along an outer circumference of the outer tube, and a distance between the two linear sections is approximately equal to a width of the annular protruding portion along a second direction perpendicular to the first direction in the radial direction.

The prefilled syringe is preferably configured that a distance between a distal surface of the finger hooking portion and a proximal surface of the pressing portion is 40 mm at maximum before discharge of the liquid-drug and is configured to be reduced to 2 to 10 mm after discharge of the liquid-drug.

The prefilled syringe is preferably configured that the syringe barrel includes a proximal extension section extending from the finger hooking portion toward the opening, an indicator portion is located on a side surface of the shaft near a proximal end, the indicator portion having a shape and a color or a pattern different from a side surface of the pressing portion, and the indicator portion is positioned proximal of the proximal extension section before discharge of the liquid-drug and is housed in the proximal extension section after discharge of the liquid-drug.

The prefilled syringe may be configured that the proximal extension section has a cut-out portion in a side opposite to a side of the first direction, the cut-out portion being formed by cutting in the proximal extension section toward the tube distal portion.

The prefilled syringe is preferably configured that the cut-out portion has an inclination such that a distance from the tube distal portion becomes shorter in the direction opposite to the first direction.

The prefilled syringe is preferably used for intradermal administration of the liquid-drug.

### Advantageous Effects of Invention

A syringe includes a gripping portion that can be held with fingers and a brim-shaped finger hooking portion protruding from the outer circumferential surface of an syringe barrel. When administrating a liquid-drug into a subject person, a user can firmly hold the gripping portion with fingers with a finger on a finger hooking portion. Holding the syringe in such a manner, the syringe can be held in a stationary manner even when a pressing portion of a plunger needs to be pushed with a high pushing force. The finger on the finger hooking portion does not disturb the push given to the pressing portion, so that the plunger can fully be pushed in with sureness to administrate a specified dose of the liquid-drug into a subject person.

The user can recognize from the finger hooking portion protruding in a single direction from the outer circumferential surface of the syringe barrel that the syringe should be held in a grabbing manner when used. Using the syringe in an incorrect manner can thus be prevented.

The syringe placed on a table, such as a medical table, will not roll because the linear section of the finger hooking portion makes contact with the table. This prevents the syringe from contamination caused by falling off the table. Thus the syringe has a high level of safety.

The syringe including the gripping portion and the finger hooking portion enables administration of a liquid-drug into a subject person with easiness, rapidness, and sureness. Therefore, the syringe is preferably used for a prefilled syringe, which is prefilled with a liquid-drug, used for administrating a specified dose of liquid-drug into each of a number of subject persons in a short period of time, such as in group vaccination.

### Brief Description of Drawings

Fig. 1 is a perspective view illustrating, with a portion cut-out portion, one embodiment of a syringe according to the present invention.
Fig. 2 is a perspective view illustrating the syringe according to the present invention just before use.
Figs. 3(a) and 3(b) are side views illustrating one embodiment of the syringe according to the present invention.
Fig. 4 is an exploded perspective view illustrating one embodiment of the syringe according to the present invention.
Fig. 5(a) is a side view of another embodiment of the syringe according to the present invention.
Fig. 5(b) is a sectional view of the embodiment of the syringe according to the present invention taken along line A-A in Fig. 5(a).
Fig. 5(c) is a sectional view of the embodiment of the syringe according to the present invention taken along line B-B in Fig. 5(a).
Fig. 5(d) is a sectional view of the embodiment of the syringe according to the present invention taken along line C-C in Fig. 5(a).
Fig. 5(e) is a partially enlarged sectional view of the embodiment of the syringe according to the present invention taken along line D-D in Fig. 5(a).
Figs. 6(a) and 6(b) are side views illustrating another embodiment of the syringe according to the present invention.
Fig. 7 is a partially exploded perspective view illustrating another embodiment of the syringe according to the present invention.
Fig. 8 is a perspective view illustrating another embodiment of the syringe according to the present invention.

### Description of Embodiments

Embodiments to carry out the present invention will be described below in detail. Note that the scope of the present invention is not limited to the embodiments.

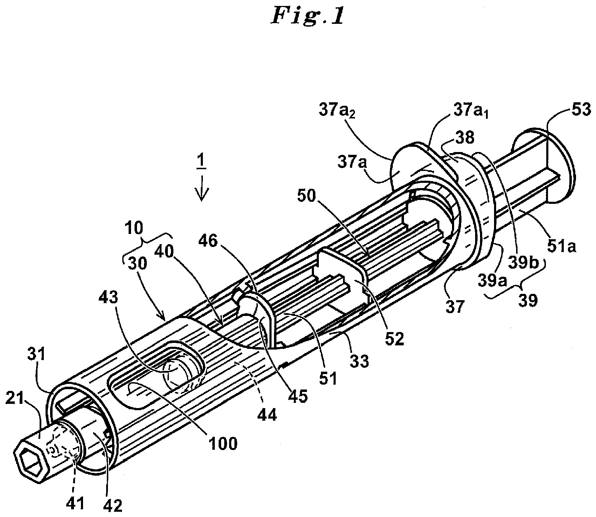

Fig. 1 is a perspective view illustrating, with a portion cut-out portion, one embodiment of a syringe according to the present invention. Fig. 1 illustrates a preferable embodiment of the syringe according to the embodiment, which is a prefilled syringe 1 storing a liquid-drug 100 for a certain purpose. The prefilled syringe 1 includes an syringe barrel 10 and a plunger 50. The syringe barrel 10 includes an outer tube 30 and a transparent syringe barrel body 40 storing a liquid-drug 100 and inserted in the outer tube 30. The most part of the plunger 50 is inserted in the syringe barrel 10. The outer tube 30, the syringe barrel body 40, and the plunger 50 are coaxially disposed.

The long plunger 50 includes a shaft 51 having a crisscross cross section, a flange 52 protruding from the side surface of the shaft 51 in a direction perpendicular to the central axis of the plunger 50, a shaft proximal portion 51a, and a disk-shaped pressing portion 53 located on the proximal end of the shaft proximal portion 51a. The shaft proximal portion 51a has a crisscross cross section larger than that of the shaft 51 to transmit the pushing force given to the pressing portion 53 straightly along the central axis of the shaft 51. The distal side of the shaft 51 is inserted in a body portion 44 from an insertion opening 46 of the syringe barrel body 40. A gasket 43 made of elastic material is in liquid-tight contact with the inner wall of the body portion 44. The proximal surface of the gasket 43 is in contact with the distal end of the plunger 50. The gasket 43 slides in the body portion 44 to discharge the liquid-drug 100.

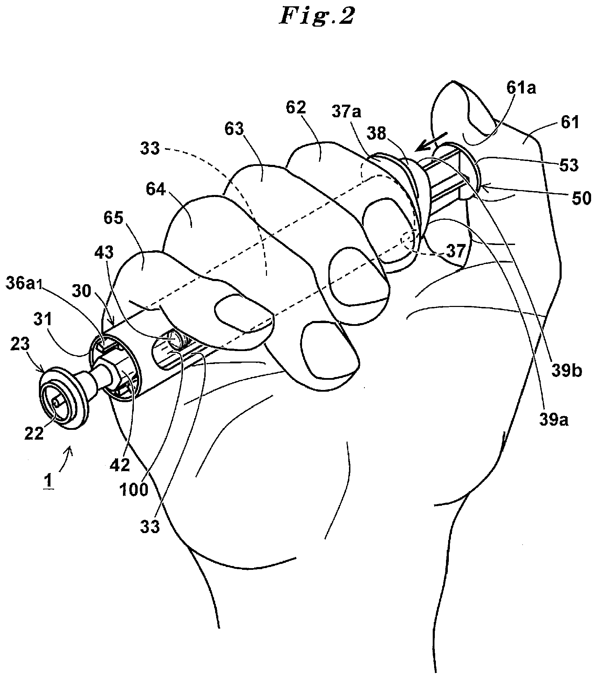

The outer tube 30 has an approximately cylindrical shape with both sides opened. The transparent syringe barrel body 40 storing the liquid-drug 100 is inserted in the outer tube 30. The outer tube 30 is made of a semitransparent material or has a pearskin-finished surface. Thus, an internal space of the outer tube 30 is not clearly visible from outside. The outer tube 30 includes a tube distal portion 31 having an opening at the distal end, a gripping portion 33 located in the middle portion continuing from the tube distal portion 31 to be held with the index finger 62, the middle finger 63, the fourth finger 64, and the fifth finger 65 (see Fig. 2), an annular protruding portion 37 located in the proximal side and having a larger diameter than the gripping portion 33, a brim-shaped finger hooking portion 37a protruding from the outer circumferential surface of the annular protruding portion 37, and a proximal extension section 38 extending in the proximal side from the annular protruding portion 37.

The finger hooking portion 37a extends in one radial direction approximately perpendicular to the central axis of the outer tube 30. The finger hooking portion 37a thus projects perpendicular to the central axis of the outer tube 30. Two parallel linear sections 37a₁ extending in this one direction and an arc section 37a₂ connecting the two linear sections form the finger hooking portion 37a.

A portion of the shaft proximal portion 51a and the pressing portion 53 protrude from an opening 39 of the proximal extension section 38. The rim of the opening 39 has an arc edge portion 39b and a cut-out portion 39a. The arc edge portion 39b forms a half the circumference of the rim in the side to the extending direction of the finger hooking portion 37a, and the cut-out portion 39a forms another half of the circumference of the rim to the opposite side. The cut-out portion 39a has an inclination such that the distance between the cut-out portion 39a and the tube distal portion 31 decreases in the direction remote from the extending direction of the finger hooking portion 37a. Thus, the arc edge portion 39b forms the proximal end of the opening 39. The annular protruding portion 37, the finger hooking portion 37a, and the proximal extension section 38 are integrally formed with the outer tube 30.

The liquid-drug 100 is stored in the internal space of the body portion 44 between the gasket 43 and a liquid-drug discharge portion 41 having a smaller diameter than the body portion 44. A cap 21 for sealing the liquid-drug discharge portion 41 is detachably attached by screwing to a luer-lock adaptor 42 located on the distal end of the syringe barrel body 40. The gasket 43 slides toward the liquid-drug discharge portion 41 by a user manually pushing the pressing portion 54 to move the plunger 50 toward the tube distal portion 31. The liquid-drug 100 is thereby discharged from the liquid-drug discharge portion 41. The plunger 50 is not connected to the gasket 43. The plunger 50 moving toward the tube distal portion 31 contacts by its distal end the proximal end of the gasket 43 to slide the gasket 43 toward the liquid-drug discharge portion 41.

The outer diameter of the pressing portion 53 is smaller than the inner diameter of the proximal extension section 38. This avoids the distal end of the pressing portion 53 interfering with the proximal end of the proximal extension section 38 when pushing in the plunger 50, so that a specified dose of the liquid-drug 100 is surely administrated.

Fig. 2 illustrates the prefilled syringe 1 just before use and just before the liquid-drug 100 is administrated into a subject person. The liquid-drug 100 is administrated into intradermal of a subject person. For example, the liquid-drug 100 includes vaccines, such as an influenza vaccine. Instead of the cap 21, a hub 23 including an injection needle 22 for administrating the liquid-drug 100 into intradermal is attached by screwing to the luer-lock adaptor 42 in a manner covering the liquid-drug discharge portion 41 (see Fig. 1). The injection needle 22 penetrates the hub 23 and communicates with the internal space of the liquid-drug discharge portion 41. The side of the index finger 62 to the thumb 61 opposes the finger hooking portion 37a. The user holds the gripping portion 33 with the index finger 62, the middle finger 63, the fourth finger 64, and the fifth finger 65. The thumb end 61a is placed on the proximal surface of the pressing portion 53. The side of the index finger 62 to the thumb 61 may be placed on the finger hooking portion 37a when using the prefilled syringe 1. The gripping portion may be held with three fingers which are the middle finger 63, the fourth finger 64, and the fifth finger 65. In this case, the side of the middle finger 63 to the thumb 61 opposes the finger hooking portion 37a.

The injection needle 22 is pierced into the skin of the subject person. The thumb 61 pushes the pressing portion 53 with high pressure toward the proximal extension section 38 (the direction indicated by a thick arrow in the figure). Even if the fingers holding the gripping portion 33 slips toward the proximal end from this state, the finger hooking portion 37a catches the finger that opposes the finger hooking portion 37a (the index finger 62 or the middle finger 63) to prevent the fingers holding the gripping portion 33 from sliding further beyond the opening 39 in the proximal side of the prefilled syringe 1. This prevents the index finger 62 or the middle finger 63 from being caught between the arc edge portion 39b of the opening 39 and the pressing portion 53 or the thumb end 61a from making contact with the side of the index finger 62 or the middle finger 63 to hinder the plunger 50 from being fully pushed in. Thus, the trouble of failing to administrate a specified dose of the liquid-drug 100 into the skin is prevented.

The finger hooking portion 37a protrudes from the outer circumferential surface of the annular protruding portion 37 in only one radial direction perpendicular to the central axis of the outer tube 30. The user can therefore recognize at a glance that the syringe is used by holding in a manner illustrated in Fig. 2. The prefilled syringe 1 has a visual appeal that allows a user to recognize the right way to use. Therefore, the prefilled syringe 1 will not be used in a wrong way, such as being used without the gripping portion 33 held. The finger hooking portion 37a has two parallel linear sections 37a₁ extending along the extending direction of the finger hooking portion 37a (see Fig. 1). The linear sections 37a₁ of the finger hooking portion 37a of the prefilled syringe 1 placed on a table, such as a medical table, makes contact with the table to prevent the prefilled syringe 1 from rolling on or falling off the table. Contamination before use is thus prevented. As described above, the prefilled syringe 1 has a high level of safety.

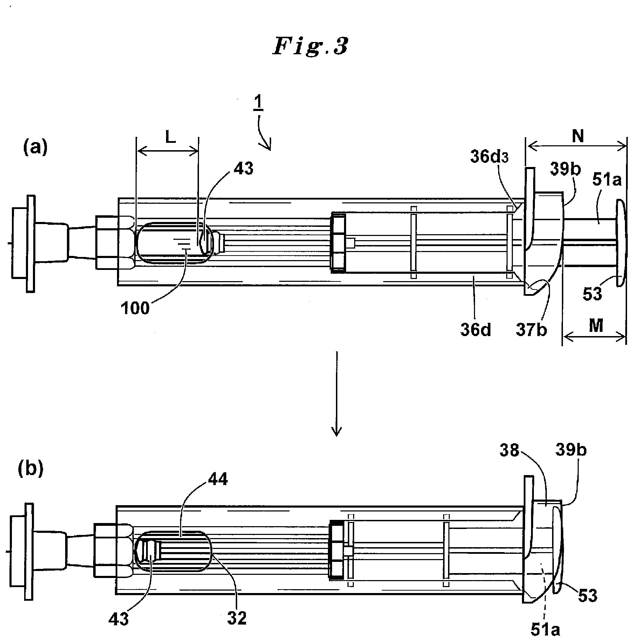

Figs. 3(a) and 3(b) are side views of the prefilled syringe 1. Fig. 3(a) illustrates an unused prefilled syringe 1. Slide distance L which the gasket 43 slides to discharge the liquid-drug 100 is approximately identical to distance M from the arc edge portion 39b, which is the proximal end of the opening 39, to the proximal surface of the pressing portion 53. As illustrated in Fig. 3(b), when the liquid-drug 100 is completely discharged, the shaft proximal portion 51a is housed in the proximal extension section 38. With the shaft proximal portion 51a having a side surface shape different from the side surface shape of the pressing portion 53, the user can see whether the shaft proximal portion 51a is projecting from the proximal extension section 38 in the proximal side and thus recognize whether the prefilled syringe 1 is used or unused. That is, the shaft proximal portion 51a functions as an indicator portion telling whether the prefilled syringe 1 is used or unused.

A square-like inspection hole 32 is located near the tube distal portion 31 of the outer tube 30, penetrating the wall of the outer tube 30 in a radial direction perpendicular to the direction in which the finger hooking portion 37a extends . Two inspection holes 32 are located at symmetric locations about the central axis of the outer tube 30. The user checks the liquid-drug 100 through the inspection holes 32 before use. On completion of the discharge of the liquid-drug 100, the inspection hole 32 visually exposes the gasket 43 that has reached the distal end of the body portion 44 and the shaft 51 in contact with the gasket 43. The user can visually check the gasket 43 and the shaft 51 through the inspection hole 32 to recognize that the liquid-drug 100 has completely been discharged.

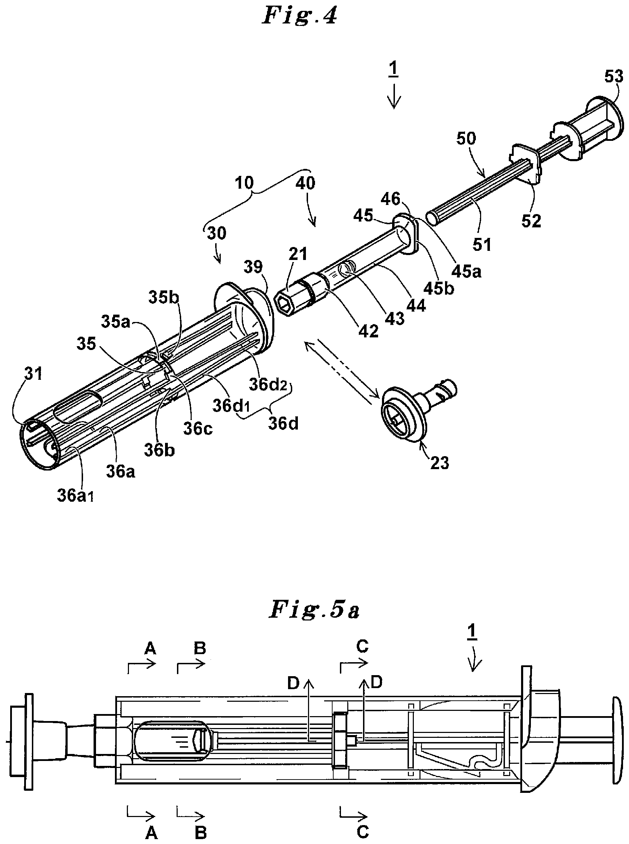

Fig. 4 is an exploded perspective view of the prefilled syringe 1. The syringe barrel body 40 includes a liquid-drug discharge portion 41 (see Fig. 1) located on the distal end, the luer-lock adaptor 42 surrounding a portion of the side surface of the liquid-drug discharge portion 41, the insertion opening 46 located on the proximal end, an syringe barrel flange 45 protruding radially outward from the rim of the insertion opening 46, and the body portion 44 having an internal space between the liquid-drug discharge portion 41 (see Fig. 1) and the insertion opening 46.

When viewed from the proximal side, the syringe barrel flange 45 has a shape with a long axis and a short axis. The syringe barrel flange 45 has a pair of long-axis side surfaces 45a on both sides in the long axis direction and a pair of short-axis side surfaces 45b on both sides in the short axis direction. Viewed from the proximal side, the outer rim of each of a pair of long-axis side surfaces 45a forms an arc about the central axis of the syringe barrel body 40. Viewed from the proximal side, the outer rim of each of a pair of short-axis side surfaces 45b forms a straight line parallel to the long axis. A pair of short-axis side surfaces 45b may be curved surfaces each having an outward convex. The luer-lock adaptor 42 is a sleeve having an external shape of a hexagonal column and an internal thread on the inner wall. The cap 21 with an external thread for sealing the liquid-drug discharge portion 41 or the hub 23 are detachably attached by screwing to the luer-lock adaptor 42.

The outer tube 30 is made of a transparent material to provide an inner space visible from external. A pair of linear guiding ribs 36d is located on a proximal portion of the inner wall of the outer tube 30 as protruding along the central axis of the outer tube 30. Two pairs of guiding ribs 36d are located at locations opposing each other with the central axis of the outer tube 30 therebetween. A pair of guiding ribs 36d extends from near the annular protruding portion 37 to the receiving portion 36b. A pair of guiding ribs 36d includes a pair of proximal guiding ribs 36d₂ and a pair of distal guiding ribs 36d₁. The proximal guiding ribs 36d₁ are located in proximal of the middle portion of the guiding ribs 36d, and the distal guiding ribs 36d₂ are located further near the receiving portion 36b than the middle portion of the guiding ribs 36d. The distance between a pair of distal guiding ribs 36d₁ increases toward the receiving portion 36b.

The distance between the opposing guiding ribs 36d is slightly greater than the width of the portion of the syringe barrel flange 45 that comes between the guiding ribs 36d but smaller than the width of the syringe barrel flange 45 along the long axis direction. This allows, during insertion of the syringe barrel body 40 from the opening 39 of the outer tube 30, the short-axis side surface 45b of the syringe barrel flange 45 to be guided by the guiding rib 36d to insert the syringe barrel body 40 into the outer tube 30 without rotating. The proximal end of the guiding rib 36d (proximal guiding rib 36d₂) reduces its height toward the opening 39, forming a guiding tapered portion 36d₃ (see Fig. 3(a)).

On the distal end of a pair of guiding ribs 36d, a pair of rotation-restricting ribs 36c protrudes from the inner wall of the outer tube 30 so as to oppose the short-axis side surface 45b of the syringe barrel flange 45 of the syringe barrel body 40 inserted in the outer tube 30. Similarly to a pair of guiding ribs 36d, two pairs of rotation-restricting ribs 36c are located at locations opposing each other with the central axis of the outer tube 30 therebetween. Near the distal end of the two pairs of rotation-restricting ribs 36c, total four receiving portions 36b are provided to protrude toward the central axis of the outer tube 30. The receiving portion 36b protrudes further than the rotation-restricting rib 36c to support the syringe barrel flange 45 of the syringe barrel body 40 inserted in the outer tube 30 by the surface of the syringe barrel flange 45 facing the distal end.

Four tilt-restricting ribs 36a protrude from the inner wall of the outer tube 30, each continuing from the receiving portion 36b to extend to the vicinity of the tube distal portion 31. Adjacent two tilt-restricting ribs 36a constitute a pair, and two pairs of tilt-restricting ribs 36a are located at locations opposing each other with the central axis of the outer tube 30 therebetween. The tilt-restricting rib 36a is allowed to contact the luer-lock adaptor 42 located on the distal end of the syringe barrel body 40. This contact restricts tilting of the syringe barrel body 40 toward the outer tube 30 when inserting the syringe barrel body 40 in the outer tube 30. The distal end of the tilt-restricting rib 36a is provided as a contact portion 36a₁ that is allowed to contact the luer-lock adaptor 42 of the syringe barrel body 40 inserted in the outer tube 30.

In the embodiment, the guiding rib 36d, the rotation-restricting rib 36c, the receiving portion 36b, and the tilt-restricting rib 36a are continuously provided, and thus as a whole, two pairs of ribs are located at locations opposing each other with the central axis of the outer tube 30 therebetween. The guiding rib 36d, the rotation-restricting rib 36c, the support protrusion 36b, and the tilt-restricting rib 36a may be provided in a non-continuous form.

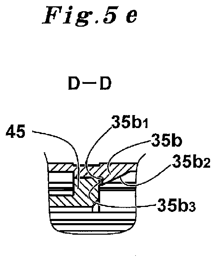

At a location shifted from the guiding rib 36d along the circumferential direction of the outer tube 30 by 90 degrees, a latching claw 35b protrudes from the inner wall of the outer tube 30. Two latching claws 35b are located at locations opposing each other with the central axis of the outer tube 30 therebetween . The latching claw 35b includes an latching claw inclined surface 35b₂ that has an inclination such that the distance from the central axis of the outer tube 30 gradually decreases toward the tube distal portion 31 and an latching surface 35b₁ that faces the tube distal portion 31 and rises in a direction approximately perpendicular to the central axis of the outer tube 30 (see Fig. 5(e)). The latching surface 35b₁ includes an latching claw protrusion 35b₃ protruding toward the tube distal portion 31 (see Fig. 6(e)). To promote elastic deformation of the portion of the outer tube 30 near each latching claw 35b in a radially outward direction, two elastic deformation promoting holes 35 are located in the outer tube 30 each near the distal end of the latching claw 35b. A wobble-restricting rib 35a extends from the distal end of each latching claw 35b toward the tube distal portion 31. Each wobble-restricting rib 35a runs across the elastic deformation promoting hole 35, so that four holes open in the outer tube 30.

When the syringe barrel body 40 is inserted in the outer tube 30, the syringe barrel flange 45 is unmovably held between the latching surface 35b₁ of the latching claw 35b and the receiving portion 36b. Each wobble-restricting rib 35a contacts a respective of the long-axis side surface 45a of the syringe barrel flange 45 to restrict the relative movement, in the long axis direction, of the syringe barrel flange 45 to the outer tube 30. The contact portion 36a₁ contacts the side surface of the luer-lock adaptor 42 to restrict the tilt of the syringe barrel body 40 toward the central axis of the outer tube 30. As described above, the prefilled syringe 1 restricts the movement of the syringe barrel body 40 in the outer tube 30.

The flange 52 protrudes from the side surface of the shaft 51. The flanges 52 contacts the inner wall of the outer tube 30 to prevent the shaft 51 of the plunger 50 from tilting toward the central axis of the syringe barrel 10 (outer tube 30). This prevents the shaft 51 from tilting toward the outer tube 30 even while pushing the pressing portion 54 of the plunger 50 with a high pushing force. The pushing force given to the pressing portion 54 thus acts on the shaft 51 along the central axis to smoothly slide the gasket 43. The flange 52 may be provided only one.

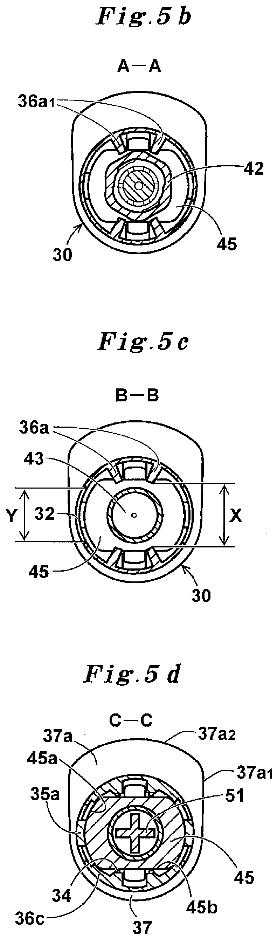

Fig. 5(a) is a side view of the prefilled syringe 1. Fig. 5(b) is a sectional view taken along line A-A in Fig. 5(a). The contact portion 36a₁ located on the distal end of the tilt-restricting rib 36a opposes the outer circumferential surface of the luer-lock adaptor 42 located on the distal end of the syringe barrel body 40. The contact portion 36a₁ protrudes toward the central axis of the outer tube 30. The contact portion 36a₁ located on the distal end of the tilt-restricting rib 36a to face the central axis of the outer tube 30 thus contacts the outer circumferential surface of the luer-lock adaptor 42 to surely restrict the tilt of the syringe barrel body 40 toward the central axis of the outer tube 30.

The distance between a pair of tilt-restricting ribs 36a and the distance between two pairs of opposing tilt-restricting ribs 36a are smaller than the outer diameter of the luer-lock adaptor 42. Thus, the contact portion 36a₁ located on the distal end of the tilt-restricting rib 36a further surely restricts the tilt of the syringe barrel body 40 toward the central axis of the outer tube 30. The entire wall of the outer tube 30 between the adjacent contact portions 36a₁ is continuously provided without any gap. This prevents the distance between the adjacent contact portions 36a₁ from widening by the luer-lock adaptor 42 making contact with the contact portion 36a₁, and thereby the tilt of the syringe barrel body 40 toward the central axis of the outer tube 30 is further surely restricted.

Fig. 5(c) illustrates a sectional view taken along line B-B in Fig. 5(a). Two inspection holes 32 are located at locations symmetric about the central axis of the outer tube 30 to open in a direction approximately perpendicular to the direction along which the two sets of tilt-restricting ribs 36a oppose each other. Distance X between the opposing two sets of tilt-restricting ribs 36a is greater than width Y of the inspection hole 32 in a cross section perpendicular to the central axis of the outer tube 30. With these dimensions, the tilt-restricting ribs 36a do not interfere in the sight when viewing the inside of the outer tube 30 along the opened direction of the inspection hole 32, and thereby the gasket 43, the liquid-drug 100, and the shaft 51 can clearly be viewed through the inspection hole 32.

Fig. 5(d) illustrates a sectional view taken along line C-C in Fig. 5(a). Each pair of rotation-restricting ribs 36c opposes a respective of a pair of long-axis side surfaces 45a of the syringe barrel flange 45. The distance between the opposing rotation-restricting ribs 36c is approximately identical to the width of the syringe barrel flange 45 that comes between the opposing rotation-restricting ribs 36c but smaller than the width of the syringe barrel flange 45 along the long axis direction. Thus, each of two sets of rotation-restricting ribs 36c contacts a respective of the long-axis side surfaces 45a of the syringe barrel flange 45 to restrict the relative rotation of the syringe barrel body 40 to the outer tube 30 and the movement of the syringe barrel flange 45 in the short axis direction.

A restricting protrusion 34 protrudes from the surface of each rotation-restricting rib 36c opposing the central axis of the outer tube 30. The restricting protrusion 34 is slightly compressingly deformed, pressed by the short-axis side surface 45b of the syringe barrel flange 45. This further surely restricts the relative rotation of the syringe barrel body 40 to the outer tube 30 and the movement of the syringe barrel flange 45 in the short axis direction.

Each wobble-restricting rib 35a opposes a respective of a pair of long-axis side surfaces 45a of the syringe barrel flange 45. The distance between the wobble-restricting ribs 35a is approximately identical to the width of the syringe barrel flange 45 along the long axis direction. Thus, each wobble-restricting rib 35a contacts a respective of the short-axis side surfaces 45b of the syringe barrel flange 45 to restrict the relative movement, in the long axis direction, of the syringe barrel flange 45 to the outer tube 30.

In this manner, the relative movement, in the long axis direction and the short axis direction of the syringe barrel flange 45, of the syringe barrel body 40 to the outer tube 30 and the relative rotation of the syringe barrel body 40 to the outer tube 30 are restricted. When viewed along the central axis of the outer tube 30, the finger hooking portion 37a includes linear sections 37a₁ parallel to the direction in which the finger hooking portion 37a protrudes from the outer wall of the outer tube 30. When inserting the syringe barrel body 40 in the outer tube 30, the direction of the outer tube 30 can be adjusted to the direction of the syringe barrel body 40 using the linear sections 37a₁ and the short-axis side surfaces 45b of the syringe barrel flange 45. The side surface of the finger hooking portion 37a includes the arc section 37a₂ connecting the two linear sections 37a₁ by extending along the outer circumference of the outer tube 30. The distance between the two linear sections 37a₁ is approximately identical to the width of the annular protruding portion 37. This allows a parts feeder in the manufacturing line to easily restrict the posture of the outer tube 30.

Fig. 5(e) illustrates a partially enlarged view of a section taken along line D-D in Fig. 5(a). The latching surface 35b₁ of the latching claw 35b includes an latching claw protrusion 35b₃ protruding toward the tube distal portion 31. The latching claw protrusion 35b₃ is pushed by a proximal surface of the syringe barrel flange 45 and is slightly compressingly deformed. As a result, there is no play between the latching claw 35b and the syringe barrel flange 45, and thereby the syringe barrel body 40 is surely prevented from moving relative to the outer tube 30 in the direction along the central axis of the outer tube 30.

The prefilled syringe 1 is manufactured as described below. The syringe barrel body 40, the luer-lock adaptor 42, and the cap 21 are formed by injection molding. The luer-lock adaptor 42 is fit on a liquid-drug discharge portion 41 so as to surroundingly cover a portion of the side surface of the liquid-drug discharge portion 41. The cap 21 is attached by screwing to the luer-lock adaptor 42. The syringe barrel body 40 is suspended in a container (not shown) to be sterilized by high pressure steam, for example.

After sterilization, a liquid-drug supply nozzle (not shown) is inserted in an insertion opening 46 of the syringe barrel body 40 to fill the syringe barrel body 40 with the intended liquid-drug 100. The gasket 43 is inserted in the syringe barrel body 40 to seal the liquid-drug 100 in a liquid-tight manner. This prevents leakage and contamination of the liquid-drug 100 in the syringe barrel body 40 while the prefilled syringe 1 is transported or stored.

The outer tube 30 formed by injection molding, for example, is prepared. The outer tube 30 provided with an inspection hole 32 created by pulling out a separable portion of a female die in a direction perpendicular to the axis of the die is prepared. The syringe barrel body 40 storing the liquid-drug 100 is inserted in the outer tube 30 so as for the short-axis side surface 45b of the syringe barrel flange 45 to oppose the guiding rib 36d. In this insertion, the direction of the outer tube 30 can easily be adjusted to the direction of the syringe barrel body 40 using the linear sections 37a₁ of the finger hooking portion 37a and the short-axis side surfaces 45b of the syringe barrel flanges 45. The short-axis side surface 45b of the syringe barrel flange 45 is guided by the guiding rib 36d to insert the syringe barrel body 40 into the outer tube 30 without rotating. The guiding tapered portion 36d₃ located on the proximal end of the guiding rib 36d (proximal guiding rib 36d₂) allows the syringe barrel flange 45 to be inserted smoothly between the opposing guiding ribs 36d.

The syringe barrel body 40 is pushed in until the syringe barrel flange 45 crosses over the latching claw 35b to be unmovably held between the receiving portion 36b and the latching claw 35b. The syringe barrel body 40 is now engaged in the outer tube 30 and disallowed to come off. Absorbing effect of the elastic deformation promoting holes 35 allows the outer tube 30 to slightly deform radially outward when the syringe barrel flange 45 crosses over the latching claw 35b. This deformation allows the syringe barrel flange 45 to cross over the latching claw 35b without compressingly deforming the latching claw 35b. The latching claw inclined surface 35b₂ of the latching claw 35b allows the syringe barrel flange 45 to easily cross over the latching claw 35b. By the insertion of the syringe barrel body 40 into the outer tube 30 as described above, the syringe barrel body 40 has a portion of the outer tube 30 extending proximally from the periphery of the insertion opening 46.

The plunger 50 including the shaft 51, the flange 52, and the pressing portion 53 formed by, for example, injection molding is prepared. The plunger 50 is inserted in the syringe barrel 10 from the opening 39 of the outer tube 30. During this insertion, the guiding tapered portion 36d₃ located on the proximal end of the guiding rib 36d (proximal guiding rib 36d₂) allows the flange 52 to be inserted smoothly between the opposing guiding ribs 36d. The plunger 50 is further pushed in until the distal surface of the shaft 51 of the plunger 50 contacts the proximal surface of the gasket 43. The manufacturing of the prefilled syringe 1 is thus completed.

The gripping portion 33 may be of any size that can be held with at least the middle finger 63, the fourth finger 64, and the fifth finger 65. The gripping portion 33 is preferably formed to have a overall length along the central axis of 70 mm or larger and an outer circumferential length of 40 to 95 mm at least at the proximal end. The gripping portion 33 can thus easily be held with at least three fingers, from the middle finger 63 to the fifth finger 65. In particular, the outer tube 30 is preferably formed in a sleeve shape having the length along the central axis from the distal end of the finger hooking portion 37a to the tube distal portion 31 of 70 mm or larger and the outer circumferential length of 40 to 95 mm. Formed in such a shape, the portion of the outer tube 30 from the distal end of the finger hooking portion 37a to the tube distal portion 31 serves as the gripping portion 33 which can easily be held at least with three fingers, from the middle finger 63 to the fifth finger 65. The length of the outer tube 30 along the central axis may be smaller than 70 mm. In such a case, the distance from the proximal end of the liquid-drug discharge portion 41 to the finger hooking portion 37a is preferably 70 mm or larger. With such dimensions, the body portion 44 of the syringe barrel body 40 is provided as a portion of the gripping portion 33, providing the overall length of the gripping portion 33 along the central axis to be 70 mm or larger.

The width of the finger hooking portion 37a along the direction perpendicular to the extending direction of the finger hooking portion 37a (the distance between two linear sections 37a₁) is preferably equal to or larger than the width of the syringe barrel 10 along the same direction as the width of the finger hooking portion 37a. Provided with such a width, the prefilled syringe 1 placed on a table is further hindered from rolling.

Preferably, the annular protruding portion 37 protrudes outward by 0.5 to 2 mm from the outer circumferential surface of the gripping portion 33, and the finger hooking portion 37a protrudes from the outer circumferential surface of the gripping portion 33 by 4 mm or larger. Provided in such a manner, the outer tube 30 can be hooked on a tool using the annular protruding portion 37 during manufacturing of the prefilled syringe 1, and the user can surely recognize at a glance that the gripping portion 33 is to be held by a grabbing manner to administrate the liquid-drug 100.

The syringe barrel body 40 is preferably formed to have the sleeve-like body portion 44 having the outer diameter of 12 mm or smaller and the overall length along the central axis of 60 mm or smaller. With the syringe barrel body 40 having such a shape, the plunger 50 can have a sufficient advance distance to discharge the liquid-drug 100 even for a small dose of stored liquid-drug 100. The user can thus feel the liquid-drug 100 being discharged by pressing the pressing portion 53 of the plunger with the thumb 61. In particular, for intradermal administration requiring a high pushing force, providing a sufficient advance distance to the plunger 50 allows the user to surely feel that the liquid-drug 100 is discharged by pushing.

The syringe barrel body 40 described above solely cannot compose the gripping portion 33 having a overall length along the central axis of 70 mm or larger and an outer circumferential length of 40 to 95 mm at least at the proximal end. Therefore, by combining with the outer tube 30 described above, the syringe barrel 10 that gives the user a feeling of the liquid-drug 100 being discharged by pushing and includes the easily-held gripping portion 33 can be provided.

The distance N between the distal surface of the finger hooking portion 37a and the proximal surface of the pressing portion 53 (see Figs. 3 (a) and 3(b)) preferably takes the maximum of 40 mm before the discharge of the liquid-drug 100 and can preferably be reduced to 2 to 10 mm after the discharge of the liquid-drug 100. Provided with such dimensions, an adult person with a hand of an average size holding the gripping portion 33 with four fingers, from the index finger 62 to the fifth finger 65, can push the pressing portion 53 of the plunger with the thumb 61. In particular, for intradermal administration requiring a high pushing force, the user can easily push the pressing portion 53 of the plunger 50 with the thumb 61. Furthermore, such a trouble can be prevented that the thumb 61 interfering, immediately before completion of the discharge of the liquid-drug 100, with the finger hooking portion 37a or the index finger 62 or the middle finger 63 on the finger hooking portion 37a to hinder the plunger 50 from being fully pushed in, resulting in failure of administrating a specified dose of the liquid-drug 100 into intradermal.

A taper 37b that gradually decreases the distance from the central axis of the outer tube 30 toward the tube distal portion 31 is preferably located on the inner wall of the annular protruding portion 37 (see Fig. 3(a)). When inserting the plunger 50 in the outer tube 30, the flange 52 slides against the taper 37b to be guided to the guiding rib 36d located further in the distal side than the annular protruding portion 37. The plunger 50 can thus be inserted smoothly in the syringe barrel 10. The taper 37b may be located at an interval or on the full circumference on the inner wall of the annular protruding portion 37. The taper 37b also allows, when inserting the syringe barrel body 40 in the outer tube 30, the syringe barrel flange 45 to be guided smoothly toward the distal side from the annular protruding portion 37. The syringe barrel body 40 can thus be inserted smoothly in the outer tube 30.

The method of using the prefilled syringe 1 will now be described. A doctor or a nurse removes the base sheet of a blister packing to take out a prefilled syringe 1 and then removes a cap 21 that has been screwed into the luer-lock adaptor 42. The film cover of a cup-shaped blister packing containing a hub 23 with an injection needle 22 is removed, and then in place of the cap 21, the hub 23 together with the cup-shaped blister packing is attached by screwing to the luer-lock adaptor 42. Since the hub 23 still packed in the cup-shaped blister packing is attached to the luer-lock adaptor 42, contamination of the injection needle 22 is avoided until immediately before the administration of the liquid-drug 100. The user sees the shaft proximal portion 51a serving as an indicator portion protruding from the opening 39 to recognize that the prefilled syringe 1 has not yet been used.

The user then removes the cup-shaped blister packing and holds the outer tube 30 with the thumb end 61a on the pressing portion 54, the fifth finger 65 to the hub 23, and the index finger 62 to the finger hooking portion 37a. The annular stabilizer 23a is pressed on a skin of a subject person where injection is to be performed, the prefilled syringe 1 is held not to sway, and then another push is given to stabilize the prefilled syringe 1. The injection needle 22 is thereby pierced into the skin of the subject person. For intradermal administration of a small dose of the liquid-drug 100 such as vaccination, in particular, the injection needle 22 is surely pierced into the skin by pressing the annular stabilizer 23a of the hub 23 onto the skin, which avoids leakage of the liquid-drug 100 from the distal end of the injection needle 22. The contact portion 36a₁ of the tilt-restricting rib 36a contacts the outer circumferential surface of the luer-lock adaptor 42 to restrict the tilt of the syringe barrel body 40 toward the central axis of the outer tube 30. In this manner, the injection needle 22 can stably be pierced into the intended portion.

Then, the pressing portion 53 is pushed by the thumb end 61a toward the tube distal portion 31 to move the plunger 50 toward the distal end. With this movement, the gasket 43 pushed by the plunger 50 slides toward the liquid-drug discharge portion 41 to discharge the liquid-drug 100 from the liquid-drug discharge portion 41. The liquid-drug 100 discharged from the liquid-drug discharge portion 41 is administrated through the injection needle 22 of the hub 23 into the subject person. The index finger 62 in contact with the finger hooking portion 37a prevents the fingers holding the outer tube 30 from moving relative to the outer tube 30 further to the proximal side. Therefore, the index finger 62 will not be caught between the arc edge portion 39a of the opening 39 and the pressing portion 53 nor the thumb end 61a will contact the side of the index finger 62, so that the plunger 50 is fully pushed in to surely inject the specified dose of the liquid-drug 100 into intradermal.

With the cut-out portion 39b located in the proximal extension section 38, the first joint of the thumb end 61a has little chance of interfering with the proximal extension section 38. The user can thus fully push in the plunger 50 without fail. Since the contact portion 36a₁ of the tilt-restricting rib 36a restricts the tilt of the syringe barrel body 40 toward the central axis of the outer tube 30 (see Fig. 2), the liquid-drug 100 can stably be administrated into the subject person.

The user gives a few seconds of push to fully push in the pressing portion 53 of the plunger 50 to complete the administration into the subject person. Since the outer diameter of the pressing portion 53 is smaller than the inner diameter of the proximal extension section 38, the distal end of the pressing portion 53 does not interfere with the proximal end of the proximal extension section 38, allowing the plunger 50 to be fully pushed in without fail. When the liquid-drug 100 has completely been discharged, the shaft proximal portion 51a, which is an indicator portion, is housed in the proximal extension section 38. The user sees the shaft proximal portion 51a not projecting in the proximal side from the proximal extension section 38 and recognizes that the prefilled syringe 1 has been used.

The shaft proximal portion 51a may have a color different from the pressing portion 53 or the proximal extension section 38. Using such a color, the unused prefilled syringe 1 can further clearly be distinguished.

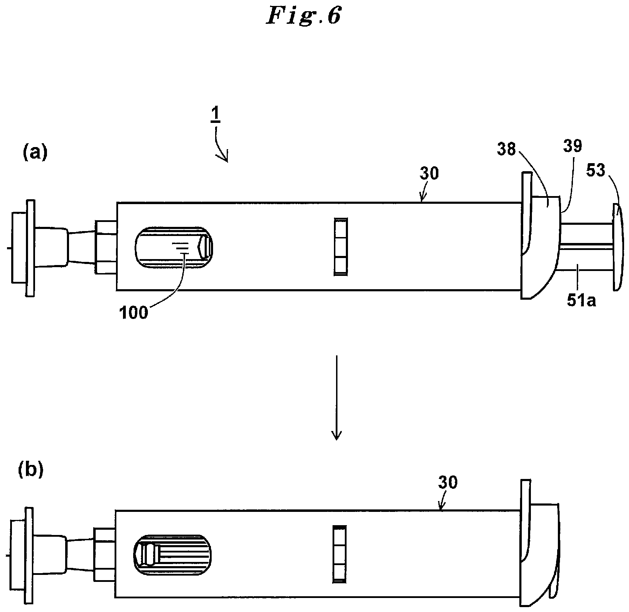

Figs. 6(a) and 6(b) are side views of another embodiment of the prefilled syringe 1. An outer tube 30 of the prefilled syringe 1 illustrated in Figs. 6(a) and 6(b) is opaque. Fig. 6(a) illustrates the unused prefilled syringe 1. A shaft proximal portion 51a serving as an indicator portion projects from the opening 39 of the opaque outer tube 30. Fig. 6(b) illustrates the used prefilled syringe 1. The shaft proximal portion 51a that has been visible before discharge of a liquid-drug 100 is concealed by the outer tube 30 and cannot be seen but for a portion at a cut-out portion 39b. The opaque outer tube 30 shows notable changes in external appearance, namely, whether the shaft proximal portion 51a serving as an indicator portion is visible, before and after the completion of discharge of the liquid-drug 100. Thus, the completion of discharge of the liquid-drug 100 can further surely be recognized by the user.

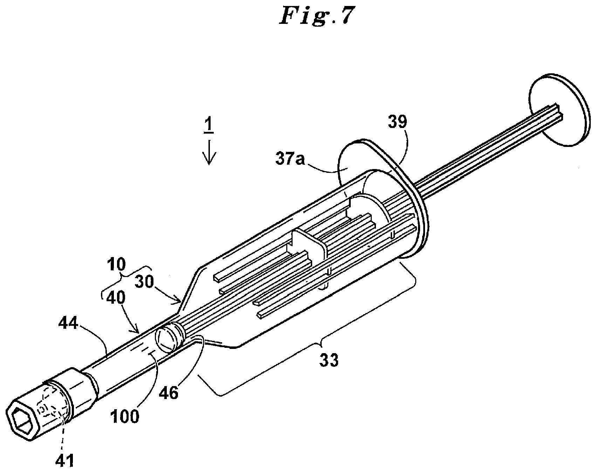

Fig. 7 is a partially exploded perspective view illustrating another embodiment of the prefilled syringe 1. An syringe barrel 10 of the prefilled syringe 1 illustrated in Fig. 7 includes an outer tube 30 and an syringe barrel body 40 integrated together. A body portion 44 of the syringe barrel body 40 has a tubular shape having an outer diameter of 12 mm or larger and a overall length along the central axis of 60 mm or smaller. The outer tube 30 extends from the periphery of an insertion opening 46 to an opening 39 to form a sleeve and includes a finger hooking portion 37a located on the outer circumferential surface at a location at least 70 mm from the proximal end of a liquid-drug discharge portion 41. The outer tube 30 includes an enlarged diameter portion 30a of which diameter expands from the periphery of the insertion opening 46 toward the opening 39. The outer tube 30 is connected to the syringe barrel body 40 via the enlarged diameter portion 30a. The section of the outer tube 30 from the proximal end of the enlarged diameter portion 30a to the distal end of the finger hooking portion 37a has an outer circumferential length of 40 to 95 mm. The prefilled syringe 1 has a gripping portion 33 including the outer tube 30 and a portion of the body portion of the syringe barrel body 40.

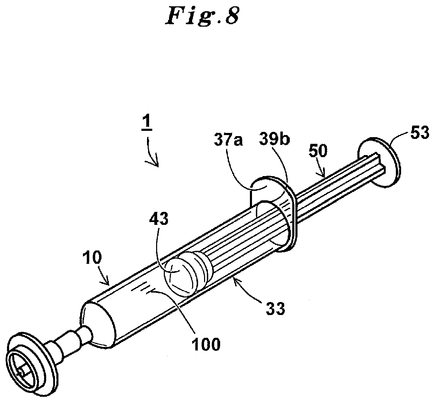

Fig. 8 is a perspective view illustrating another embodiment of the prefilled syringe 1. The prefilled syringe 1 includes an syringe barrel 10 and a plunger 50 and does not include an outer tube 30.

A finger hooking portion 37a protrudes at the proximal end of the syringe barrel 10. In the embodiment, a gripping portion 33 is held with at least a middle finger 63, a fourth finger 64, and a fifth finger 65 with the finger hooking portion 37a making contact with the side of an index finger 62 or the middle finger 63. A thumb end 61a contacts the proximal surface of a pressing portion 53. The pressing portion 53 is pushed by the thumb end 61a. Using the prefilled syringe 1 in such a manner prevents the index finger 62 being caught between the arc edge portion 39b of the opening 39 and the pressing portion 53 or the thumb end 61a contacting the side of the index finger 62 to hinder the plunger 50 from being fully pushed in, which might cause failure of administrating a specified dose of the liquid-drug 100 into a skin.

Other than pharmaceutical for treating rheumatism, the liquid-drug 100 stored in the syringe barrel body 40 may be a bio-pharmaceutical such as vaccine for preventing, for example, influenza, tetanus, streptococcus pneumoniae, poliomyelitis, Japanese encephalitis, rubella, measles, yellow fever, HIV, hepatitis, chickenpox, rabies, rotavirus, mumps, or uterine cervix cancer, MQ vaccine, DT vaccine, and DPT vaccine. Examples other than vaccines include: saccharide infusion solutions such as glucose; electrolyte regulating infusion solutions such as sodium chloride and potassium lactate; contrast media; steroids; protease inhibitors; fat emulsions; antibiotics; anticancer agents; heparin calcium; anesthetics; and antibody preparations.

The plunger 50 is usually pushed with a pushing force below 5 N to subcutaneously administrate the liquid-drug using the prefilled syringe. When the plunger 50 is pushed with a pushing force of 5 N or larger, a user such as a doctor pushes the plunger 50 with a pushing force higher than usual. While a push is given to the pressing portion 53 of the plunger 50 in such a case, a finger hooking portioning the gripping portion 33 may easily slip toward the distal end. As a result, the index finger 62 may be caught between the arc edge portion 39b of the opening 39 and the pressing portion 53 or the thumb end 61a may contact the side of the index finger 62 to hinder the plunger 50 from being fully pushed in, which increases the risk of a specified dose of the liquid-drug 100 not being administrated. Thus, the prefilled syringe 1 is preferably used when the plunger 50 is to be pushed with a pushing force of 5 N or larger. In particular, the prefilled syringe 1 is preferably used for administrating the liquid-drug 100 into intradermal, which requires a pushing force of approximately 15 N or larger.

The prefilled syringe 1 may be sterilized by autoclave sterilization or sterilization using any one of ethylene oxide gas, γ-ray, and electron ray.

Materials of the cap 21, the hub 23, the outer tube 30, the syringe barrel body 40, and the plunger 50 are each selected from a view point of, for example, chemical resistance, barrier properties against gas and bacteria, and safety for living bodies. For example, such materials include: polyolefin resin such as polyethylene, polypropylene, and cyclic polyolefin; polystyrene; polycarbonate; polyester such as polyethylene terephthalate; and polyamide. If sterilization is performed in an autoclave, in particular, a high thermal resistant resin, for example, polypropylene or polycarbonate is preferably used as such material. As the material of the syringe barrel body 40, cyclic olefin homopolymer or cyclic olefin copolymer is preferably used, since such resins have high transparency allowing the liquid-drug 100 stored in the tube to be viewed from outside, little interaction with the liquid-drug 100, and sufficient stiffness that prevents bending or crushing under firm holding and hard pushing during administration of the liquid-drug 100. Components of the prefilled syringe 1 may be formed by injection molding or other methods, such as blow molding and thermal molding.

The material of the gasket 43 is selected from the same view point as described above. For example, thermoplastic elastomer of olefin-base, polyurethane-base, polyester-base, polyamide-base, or styrene-base or rubber materials such as natural rubber, isoprene rubber, butadiene rubber, styrene-butadiene rubber, and silicone rubber may be used.

### Industrial Applicability

A prefilled syringe according to the present invention is used for administrating a liquid-drug into a subject person.

### Reference Signs List

- 1: prefilled syringe
- 10: syringe barrel
- 21: cap
- 22: injection needle
- 23: hub
- 23a: annular stabilizer
- 30: outer tube
- 30a: enlarged diameter portion
- 30b: through hole
- 31: tube distal portion
- 32: inspection hole
- 33: gripping portion
- 34: restricting protrusion
- 35: elastic deformation promoting hole
- 35a: wobble-restricting rib
- 35b: latching claw
- 35b₁: latching surface
- 35b₂: latching claw inclined surface
- 35b₃: latching claw protrusion
- 36a: tilt-restricting rib
- 36a₁: contact portion
- 36b: receiving portion
- 36c: rotation-restricting rib
- 36d: guiding rib
- 36d₁: distal guiding rib
- 36d₂: proximal guiding rib
- 36d₃: guiding tapered portion
- 37: annular protruding portion
- 37a: finger hooking portion
- 37a₁: linear section
- 37a₂: arc section
- 37b: taper
- 38: proximal extension section
- 39: opening
- 39a: arc edge portion
- 39b: cut-out portion
- 40: syringe barrel body
- 41: liquid-drug discharge portion
- 42: luer-lock adaptor
- 43: gasket
- 44: body portion
- 45: syringe barrel flange
- 45a: long-axis side surface
- 45b: short-axis side surface
- 46: insertion opening
- 50: plunger
- 51: shaft
- 51a: shaft proximal portion
- 52: flange
- 53: pressing portion
- 61: thumb
- 61a: thumb end
- 62: index finger
- 63: middle finger
- 64: fourth finger
- 65: fifth finger
- 100: liquid-drug
- L, M, N: distance

## Claims

1. A syringe (1) comprising:
a syringe barrel (10) including:
a liquid-drug discharge portion (41) on a distal end of the barrel (10),
an opening (39) on a proximal end of the barrel (10), and
a body portion (44) for storing a liquid-drug (100);
a gasket (43) inserted in the syringe barrel (10); and
a plunger (50) including:
a shaft (51) inserted in the syringe barrel (10) from the opening and:
a pressing portion (53) located on a proximal end of the shaft (51), the pressing portion (53) being pushable by a thumb (61) to slide the gasket (43) toward the liquid-drug discharge portion (41), wherein
the syringe barrel (10) is **characterised in that** it further includes:
a brim-shaped finger hooking portion (37a) protruding from an outer circumferential surface of the syringe barrel (10) so as to extend in a first direction, which is a radial direction approximately perpendicular to a central axis of the syringe barrel (10), the finger hooking portion (37a) being configured to be hooked with a finger; and
a sleeve-shaped gripping portion (33) that is located between a distal end of the finger hooking portion (37a) and a proximal end of the liquid-drug discharge portion (41) and is configured to be held with at least a middle finger (63), a fourth finger (64), and a fifth finger (65),
wherein the syringe barrel (10) includes an annular protruding portion (37) between the gripping portion (33) and the opening (39), the annular protruding portion (37) protruding outward from an outer circumferential surface of the gripping portion (33) by 0.5 to 2 mm in a direction approximately perpendicular to the central axis, and
wherein the finger hooking portion (37a) protrudes from an outer circumferential surface of the annular protruding portion (37) by at least 4 mm in only one radial direction perpendicular to the central axis.

2. The syringe (1) according to claim 1, wherein the gripping portion (33) has an overall length of at least 70 mm along the central axis and an outer circumferential length of 40 to 95 mm at least at a proximal portion of the gripping portion (33).

3. The syringe (1) according to any one of claims 1 to 2, wherein a width of the finger hooking portion (37a) along a second direction perpendicular to the first direction in the radial direction is equal to or larger than a width of the syringe barrel (10) along the second direction.

4. The syringe (1) according to any one of claims 1 to 3, wherein the syringe barrel (10) further includes:
an syringe barrel body (40) including the liquid-drug discharge portion (41) at a distal end of syringe barrel body (40), an insertion opening (46) opened at a proximal end of syringe barrel body (40), and the body portion (44) that is located between the liquid-drug discharge portion (41) and the insertion opening (46) and has a form of a tube with a maximum outer diameter of 12 mm and a maximum overall length of 60 mm along the central axis; and
an outer tube (30) extending at least from a periphery of the insertion opening (46) to the opening (39) so as to form a sleeve, wherein the outer tube (30) includes the finger hooking portion (37a), and the finger hooking portion (37a) is located at least 70 mm from a proximal end of the liquid-drug discharge portion (41) and protrudes from an outer circumferential surface of the outer tube (30) in the first direction, and wherein the outer tube (30) has an outer circumferential length of 40 to 95 mm at least near a distal end of the finger hooking portion (37a), and wherein the outer tube (30) constitutes at least a proximal portion of the gripping portion (33).

5. The syringe (1) according to any one of claims 1 to 4, used for intradermal administration of the liquid-drug (100).

6. A prefilled syringe (1) comprising:
the syringe (1) according to any one of claims 1 to 5;
a cap (21) detachably attached to the syringe barrel (10) to seal the liquid-drug discharge portion (41); and
a liquid-drug (100) that is stored between the liquid-drug discharge portion (41) and the gasket (43) and is dischargeable through the liquid-drug discharge portion (41) by sliding of the gasket (43).

7. The prefilled syringe (1) according to claim 6, wherein:
the syringe barrel (10) includes:
an syringe barrel body (40) including the liquid-drug discharge portion (41) at a distal end of syringe barrel body (40), an insertion opening (46) opened at a proximal end of syringe barrel body (40), the body portion (44), and an syringe barrel flange (45) that protrudes from a rim of the insertion opening (46), and wherein the body portion (44) is located between the liquid-drug discharge portion (41) and the insertion opening (46) and has a form of a tube with a maximum outer diameter of 12 mm and a maximum overall length along the central axis of 60 mm; and
an outer tube (30) including a tube distal portion (31) opened at a distal end of the outer tube (30) and the opening (39) at a proximal end of the outer tube (30), wherein the outer tube (30) is formed of a sleeve in which the syringe barrel body (40) is configured to be inserted, wherein the outer tube (30) includes the finger hooking portion (37a) that is located at least 70 mm from a proximal end of the liquid-drug discharge portion (41) and protrudes from an outer circumferential surface of the outer tube (30) in the first direction, wherein the outer tube (30) has an outer circumferential length of 40 to 95 mm at least near a distal end of the finger hooking portion (37a), and wherein the outer tube (30) unmovably holds the syringe barrel flange (45) to support the syringe barrel body (40) inside the outer tube (30), and
the outer tube (30) constitutes at least a proximal portion of the gripping portion (33).

8. The prefilled syringe (1) according to claim 7, wherein the outer tube (30) extends from the rim of the insertion opening (46) toward the distal end of the syringe barrel body (40) so as to form a sleeve surrounding a side surface of the body portion (44), the outer circumferential length of the outer tube (30) having a range of 40 to 95 mm.

9. The prefilled syringe (1) according to claim 8, wherein the outer tube (30) includes an inspection hole (32) penetrating a wall of the outer tube (30) in a second direction perpendicular to the first direction in the radial direction, the body portion (44) being visually exposed through the inspection hole (32).

10. The prefilled syringe (1) according to claim 9, wherein:
the outer tube (30) includes two pairs of ribs (36d) at locations opposing each other with the central axis therebetween, the ribs (36d) in each pair protruding from one side of an inner wall of the outer tube (30) and linearly along the central axis,
each of the two pair of ribs (36d) includes a contact portion and a support portion, the contact portion configured to contact with an outer circumferential surface of a distal portion of the syringe barrel body (40) to prevent the syringe barrel body (40) from tilting toward the outer tube (30), the support portion supporting a distal surface of the syringe barrel flange (45), and
a distance between the two pairs of ribs (36d) opposing each other is greater than a width of the inspection hole (32) along the first direction.

11. The prefilled syringe (1) according to any one of claims 7 to 10, wherein:
the syringe barrel flange (45) has an outer profile having a long axis and a short axis perpendicular to the long axis when viewed from a proximal side of syringe barrel flange (45), the syringe barrel flange (45) including a pair of long-axis side surfaces provided at both ends in the long axis direction and a pair of short-axis side surfaces provided at both ends in the short axis direction, and
the outer tube (30) includes rotation-restricting ribs (36c) each protruding from the inner wall of the outer tube (30) toward the short-axis side surface of the syringe barrel flange (45), each rotation-restricting rib (36c) being configured to contact a respective the short-axis side surface to restrict the syringe barrel body (40) from rotating relative to the outer tube (30).

12. The prefilled syringe (1) according to claim 11, wherein the syringe barrel body (40) includes a luer-lock adaptor (42) that surrounds a side surface of the liquid-drug discharge portion (41) and is configured to be screwably attached to a hub (23) with an injection needle (22) for administrating the liquid-drug (100).

13. The prefilled syringe (1) according to claim 11 or 12, wherein:
the outer tube (30) includes the annular protruding portion (37) located between the gripping portion (33) and the opening (39), the annular protruding portion (37) protruding outward from an outer circumferential surface of the outer tube (30) by 0.5 to 2 mm,
the finger hooking portion (37a) extends in the first direction so as to connect to an outer circumferential surface of the annular protruding portion (37),
a side surface of the finger hooking portion (37a) includes two parallel linear sections (37a1) extending along the first direction and an arc section (37a2) connecting the two linear sections (37a1) by extending along an outer circumference of the outer tube (30), and
a distance between the two linear sections (37a1) is approximately equal to a width of the annular protruding portion (37) along a second direction perpendicular to the first direction in the radial direction.

14. The prefilled syringe (1) according to any one of claims 6 to 13, wherein a distance between a distal surface of the finger hooking portion (37a) and a proximal surface of the pressing portion (53) is 40 mm at maximum before discharge of the liquid-drug (100) and is configured to be reduced to 2 to 10 mm after discharge of the liquid-drug (100).

## Patentansprüche

1. Spritze (1), umfassend:
einen Spritzenzylinder (10), umfassend:
einen Abgabeabschnitt (41) für ein flüssiges Arzneimittel an einem distalen Ende des Zylinders (10),
eine Öffnung (39) an einem proximalen Ende des Zylinders (10), und
ein Körperabschnitt (44) zur Speicherung eines flüssigen Arzneimittels (100);
eine Dichtung (43), die in den Spritzenzylinder (10) eingesetzt ist; und
einen Kolben (50), umfassend:
einen Schaft (51), der von der Öffnung her in den Spritzenzylinder (10) eingesetzt ist; und:
einen Druckabschnitt (53), der an einem proximalen Ende des Schafts (51) angeordnet ist, wobei der Druckabschnitt (53) durch einen Daumen (61) geschoben werden kann, um die Dichtung (43) in Richtung des Abgabeabschnitts (41) für ein flüssiges Arzneimittel zu schieben, wobei der Spritzenzylinder (10) **dadurch gekennzeichnet ist, dass** er ferner umfasst:
einen krempenförmigen Finger-Einhakabschnitt (37a), der von einer äußeren Umfangsfläche des Spritzenzylinders (10) so vorsteht, dass er sich in einer ersten Richtung erstreckt, welche eine radiale Richtung ist, die ungefähr senkrecht zu einer Mittelachse des Spritzenzylinders (10) steht, wobei der Finger-Einhakabschnitt (37a) so konfiguriert ist, dass er mit einem Finger eingehakt werden kann; und
einen hülsenförmigen Greifabschnitt (33), der zwischen einem distalen Ende des Finger-Einhakabschnitts (37a) und einem proximalen Ende des Abgabeabschnitts (41) für ein flüssiges Arzneimittel angeordnet ist und so konfiguriert ist, dass er mit mindestens einem Mittelfinger (63), einem vierten Finger (64) und einem fünften Finger (65) gehalten wird,
wobei der Spritzenzylinder (10) einen ringförmig vorstehenden Abschnitt (37) zwischen dem Greifabschnitt (33) und der Öffnung (39) aufweist, wobei der ringförmig vorstehende Abschnitt (37) von einer äußeren Umfangsfläche des Greifabschnitts (33) um 0,5 bis 2 mm in einer Richtung, die ungefähr senkrecht zu der Mittelachse ist, nach außen vorsteht, und
wobei der Finger-Einhakabschnitt (37a) von einer äußeren Umfangsfläche des ringförmigen vorstehenden Abschnitts (37) um mindestens 4 mm in nur einer radialen Richtung senkrecht zu der Mittelachse vorsteht.

2. Spritze (1) nach Anspruch 1, wobei der Greifabschnitt (33) eine Gesamtlänge von mindestens 70 mm entlang der Mittelachse und eine äußere Umfangslänge von 40 bis 95 mm zumindest an einem proximalen Abschnitt des Greifabschnitts (33) aufweist.

3. Spritze (1) nach einem der Ansprüche 1 bis 2, wobei eine Breite des Finger-Einhakabschnitts (37a) entlang einer zweiten Richtung, die senkrecht zur ersten Richtung ist, in der radialen Richtung gleich oder größer als eine Breite des Spritzenzylinders (10) entlang der zweiten Richtung ist.

4. Spritze (1) nach einem der Ansprüche 1 bis 3, wobei der Spritzenzylinder (10) ferner umfasst:
einen Spritzenzylinderkörper (40), der den Abgabeabschnitt (41) für ein flüssiges Arzneimittel an einem distalen Ende des Spritzenzylinderkörpers (40), eine Einführöffnung (46), die an einem proximalen Ende des Spritzenzylinderkörpers (40) geöffnet ist, und den Körperabschnitt (44), der zwischen dem Abgabeabschnitt (41) für ein flüssiges Arzneimittel und der Einführöffnung (46) angeordnet ist und die Form eines Rohrs mit einem maximalen Außendurchmesser von 12 mm und einer maximalen Gesamtlänge von 60 mm entlang der Mittelachse aufweist, umfasst; und
ein Außenrohr (30), das sich mindestens von einem Umfang der Einführöffnung (46) zu der Öffnung (39) erstreckt, um eine Hülse zu bilden, wobei das Außenrohr (30) den Finger-Einhakabschnitt (37a) umfasst und der Finger-Einhakabschnitt (37a) mindestens 70 mm von einem proximalen Ende des Abgabeabschnitts (41) für ein flüssiges Arzneimittel angeordnet ist und von einer äußeren Umfangsfläche des Außenrohrs (30) in der ersten Richtung vorsteht, und wobei das Außenrohr (30) eine äußere Umfangslänge von 40 bis 95 mm mindestens in der Nähe eines distalen Endes des Finger-Einhakabschnitts (37a) aufweist und wobei das Außenrohr (30) mindestens einen proximalen Abschnitt des Greifabschnitts (33) bildet.

5. Spritze (1) nach einem der Ansprüche 1 bis 4, die zur intradermalen Verabreichung des flüssigen Arzneimittels (100) verwendet wird.

6. Vorgefüllte Spritze (1), umfassend:
die Spritze (1) nach einem der Ansprüche 1 bis 5;
eine Kappe (21), die abnehmbar an dem Spritzenzylinder (10) befestigt ist, um den Abgabeabschnitt (41) für ein flüssiges Arzneimittel zu verschließen; und
ein flüssiges Arzneimittel (100), das zwischen dem Abgabeabschnitt (41) für ein flüssiges Arzneimittel und der Dichtung (43) gespeichert ist und durch den Abgabeabschnitt (41) für ein flüssiges Arzneimittel durch Gleiten der Dichtung (43) abgegeben werden kann.

7. Vorgefüllte Spritze (1) nach Anspruch 6, wobei:
der Spritzenzylinder (10) umfasst:
einen Spritzenzylinderkörper (40), der den Abgabeabschnitt (41) für ein flüssiges Arzneimittel an einem distalen Ende des Spritzenzylinderkörpers (40), eine Einführöffnung (46), die an einem proximalen Ende des Spritzenzylinderkörpers (40) geöffnet ist, den Körperabschnitt (44) und einen Spritzenzylinderflansch (45) umfasst, der von einem Rand der Einführöffnung (46) vorsteht, und wobei der Körperabschnitt (44) zwischen dem Abgabeabschnitt (41) für ein flüssiges Arzneimittel und der Einführöffnung (46) angeordnet ist und die Form eines Rohrs mit einem maximalen Außendurchmesser von 12 mm und einer maximalen Gesamtlänge von 60 mm entlang der Mittelachse aufweist, und
ein Außenrohr (30), das einen distalen Rohrabschnitt (31), der an einem distalen Ende des Außenrohrs (30) geöffnet ist, und die Öffnung (39) an einem proximalen Ende des Außenrohrs (30) aufweist, wobei das Außenrohr (30) aus einer Hülse gebildet ist, in welche der Spritzenzylinderkörper (40) zum Einführen konfiguriert ist, wobei das Außenrohr (30) den Finger-Einhakabschnitt (37a) aufweist, der mindestens 70 mm von einem proximalen Ende des Abgabeabschnitts (41) für ein flüssiges Arzneimittel entfernt angeordnet ist und von einer äußeren Umfangsfläche des Außenrohrs (30) in der ersten Richtung vorsteht, wobei das Außenrohr (30) eine äußere Umfangslänge von 40 bis 95 mm mindestens in der Nähe eines distalen Endes des Finger-Einhakabschnitts (37a) aufweist, und wobei das Außenrohr (30) den Spritzenzylinderflansch (45) unbeweglich hält, um den Spritzenzylinderkörper (40) innerhalb des Außenrohrs (30) zu stützen, und
das Außenrohr (30) mindestens einen proximalen Abschnitt des Greifabschnitts (33) bildet.

8. Vorgefüllte Spritze (1) nach Anspruch 7, wobei sich das Außenrohr (30) von dem Rand der Einführöffnung (46) in Richtung des distalen Endes des Spritzenzylinderkörpers (40) erstreckt, um eine Hülse zu bilden, die eine Seitenfläche des Körperabschnitts (44) umgibt, wobei die äußere Umfangslänge des Außenrohrs (30) einen Bereich von 40 bis 95 mm aufweist.

9. Vorgefüllte Spritze (1) nach Anspruch 8, wobei das Außenrohr (30) eine Überprüfungsöffnung (32) aufweist, die eine Wand des Außenrohrs (30) in einer zweiten Richtung, die senkrecht zu der ersten Richtung ist, in der radialen Richtung durchdringt, wobei der Körperabschnitt (44) durch die Überprüfungsöffnung (32) visuell freigelegt ist.

10. Vorgefüllte Spritze (1) nach Anspruch 9, wobei:
das Außenrohr (30) zwei Paare von Rippen (36d) an einander gegenüberliegenden Stellen mit der Mittelachse dazwischen aufweist, wobei die Rippen (36d) in jedem Paar von einer Seite einer Innenwand des Außenrohrs (30) und linear entlang der Mittelachse vorstehen,
jedes der beiden Paare von Rippen (36d) einen Kontaktabschnitt und einen Stützabschnitt umfasst, wobei der Kontaktabschnitt so konfiguriert ist, dass er mit einer äußeren Umfangsfläche eines distalen Abschnitts des Spritzenzylinderkörpers (40) in Kontakt kommt, um zu verhindern, dass der Spritzenzylinderkörper (40) in Richtung des Außenrohrs (30) kippt, wobei der Stützabschnitt eine distale Fläche des Spritzenzylinderflansches (45) stützt, und
ein Abstand zwischen den beiden einander gegenüberliegenden Paaren von Rippen (36d) größer ist als eine Breite der Überprüfungsöffnung (32) in der ersten Richtung.

11. Vorgefüllte Spritze (1) nach einem der Ansprüche 7 bis 10, wobei:
der Spritzenzylinderflansch (45) ein Außenprofil mit einer Längsachse und einer Kurzachse senkrecht zu der Längsachse aufweist, wenn er von einer proximalen Seite des Spritzenzylinderflansches (45) aus betrachtet wird, wobei der Spritzenzylinderflansch (45) ein Paar Längsachsen-Seitenflächen, die an beiden Enden in der LängsachsenRichtung vorgesehen sind, und ein Paar Kurzachsen-Seitenflächen, die an beiden Enden in der Kurzachsen-Richtung vorgesehen sind, umfasst, und
das Außenrohr (30) drehungseinschränkende Rippen (36c) aufweist, die jeweils von der Innenwand des Außenrohrs (30) in Richtung der Kurzachsen-Seitenfläche des Spritzenzylinderflansches (45) vorstehen, wobei jede drehungseinschränkende Rippe (36c) so konfiguriert ist, dass sie mit einer entsprechenden Kurzachsen-Seitenfläche in Kontakt kommt, um den Spritzenzylinderkörper (40) an einer Drehung relativ zu dem Außenrohr (30) zu hindern.

12. Vorgefüllte Spritze (1) nach Anspruch 11, wobei der Spritzenzylinderkörper (40) einen Luer-Lock-Adapter (42) aufweist, der eine Seitenfläche des Abgabeabschnitts (41) für ein flüssiges Arzneimittel umgibt und so konfiguriert ist, dass er an einer Nabe (23) mit einer Injektionsnadel (22) zur Verabreichung des flüssigen Arzneimittels (100) schraubbar befestigt werden kann.

13. Vorgefüllte Spritze (1) nach Anspruch 11 oder 12, wobei:
das Außenrohr (30) den ringförmigen vorstehenden Abschnitt (37) umfasst, der zwischen dem Greifabschnitt (33) und der Öffnung (39) angeordnet ist, wobei der ringförmige vorstehende Abschnitt (37) von einer äußeren Umfangsfläche des Außenrohrs (30) um 0,5 bis 2 mm nach außen vorsteht,
der Finger-Einhakabschnitt (37a) sich in die erste Richtung so erstreckt, dass er sich mit einer äußeren Umfangsfläche des ringförmigen vorstehenden Abschnitts (37) verbindet,
eine Seitenfläche des Finger-Einhakabschnitts (37a) zwei parallele lineare Abschnitte (37a1), die sich entlang der ersten Richtung erstrecken, und einen Bogenabschnitt (37a2) umfasst, der die beiden linearen Abschnitte (37a1) verbindet, indem er sich entlang eines Außenumfangs des Außenrohrs (30) erstreckt, und
ein Abstand zwischen den beiden linearen Abschnitten (37a1) ungefähr gleich einer Breite des ringförmigen vorstehenden Abschnitts (37) entlang einer zweiten Richtung, die senkrecht zu der ersten Richtung ist, in der radialen Richtung ist.

14. Vorgefüllte Spritze (1) nach einem der Ansprüche 6 bis 13, wobei ein Abstand zwischen einer distalen Fläche des Finger-Einhakabschnitts (37a) und einer proximalen Fläche des Druckabschnitts (53) vor der Abgabe des flüssigen Arzneimittels (100) maximal 40 mm beträgt und so konfiguriert ist, dass er nach der Abgabe des flüssigen Arzneimittels (100) auf 2 bis 10 mm verringert wird.

## Revendications

1. Seringue (1) comprenant :
un cylindre de seringue (10) comprenant :
une partie de sortie de médicament liquide (41) sur une extrémité distale du cylindre (10),
une ouverture (39) sur une extrémité proximale du cylindre (10), et
une partie corps (44) pour stocker un médicament liquide (100) ;
un joint d'étanchéité (43) inséré dans le cylindre de seringue (10) ; et
un piston (50) comprenant :
un axe (51) inséré dans le cylindre de seringue (10) à partir de l'ouverture ; et
une partie de pression (53) située sur une extrémité proximale de l'axe (51), la partie de pression (53) pouvant être poussée par un pouce (61) pour faire glisser le joint d'étanchéité (43) vers la partie de sortie de médicament liquide (41), où
le cylindre de seringue (10) est **caractérisé en ce que** qu'il comprend en outre :
une partie d'accrochage de doigt en forme de rebord (37a) dépassant d'une surface circonférentielle extérieure du cylindre de seringue (10) de manière à s'étendre dans une première direction, qui est une direction radiale approximativement perpendiculaire à un axe central du cylindre de seringue (10), la partie d'accrochage de doigt (37a) étant conçue pour être accrochée avec un doigt ; et
une partie de préhension en forme de manchon (33) qui est placée entre une extrémité distale de la partie d'accrochage de doigt (37a) et une extrémité proximale de la partie de sortie de médicament liquide (41) et est conçue pour être tenue avec au moins le majeur (63), le quatrième doigt (64), et le cinquième doigt (65),
où le cylindre de seringue (10) comprend une partie saillante annulaire (37) entre la partie de préhension (33) et l'ouverture (39), la partie saillante annulaire (37) faisant saillie vers l'extérieur depuis une surface circonférentielle extérieure de la partie de préhension (33) de 0,5 à 2 mm dans une direction approximativement perpendiculaire à l'axe central, et
où la partie d'accrochage de doigt (37a) fait saillie depuis une surface circonférentielle extérieure de la partie saillante annulaire (37) d'au moins 4 mm dans une seule direction radiale perpendiculaire à l'axe central.

2. Seringue (1) selon la revendication 1, dans laquelle la partie de préhension (33) a une longueur totale d'au moins 70 mm le long de l'axe central et une longueur circonférentielle extérieure de 40 à 95 mm au moins au niveau d'une partie proximale de la partie de préhension (33).

3. Seringue (1) selon l'une quelconque des revendications 1 à 2, dans laquelle une largeur de la partie d'accrochage de doigt (37a) le long d'une seconde direction perpendiculaire à la première direction dans la direction radiale est supérieure ou égale à une largeur du cylindre de seringue (10) le long de la seconde direction.

4. Seringue (1) selon l'une quelconque des revendications 1 à 3, dans laquelle le cylindre de seringue (10) comprend en outre :
un corps de cylindre de seringue (40) comprenant la partie de sortie de médicament liquide (41) à une extrémité distale du corps de cylindre de seringue (40), une ouverture d'insertion (46) ouverte à une extrémité proximale du corps de cylindre de seringue (40), et la partie corps (44) qui est située entre la partie de sortie de médicament liquide (41) et l'ouverture d'insertion (46) et a une forme de tube avec un diamètre extérieur maximum de 12 mm et une longueur totale maximum de 60 mm le long de l'axe central ; et
un tube extérieur (30) s'étendant au moins depuis une périphérie de l'ouverture d'insertion (46) jusqu'à l'ouverture (39) de manière à former un manchon, où le tube extérieur (30) comprend la partie d'accrochage de doigt (37a), et la partie d'accrochage de doigt (37a) est située à au moins 70 mm d'une extrémité proximale de la partie de sortie de médicament liquide (41) et fait saillie depuis une surface circonférentielle extérieure du tube extérieur (30) dans la première direction, et où le tube extérieur (30) a une longueur circonférentielle extérieure de 40 à 95 mm au moins près d'une extrémité distale de la partie d'accrochage de doigt (37a), et où le tube extérieur (30) constitue au moins une partie proximale de la partie de préhension (33).

5. Seringue (1) selon l'une quelconque des revendications 1 à 4, utilisée pour l'administration intradermique du médicament liquide (100).

6. Seringue pré-remplie (1) comprenant :
la seringue (1) selon l'une quelconque des revendications 1 à 5 ;
un bouchon (21) fixé de façon détachable au cylindre de seringue (10) pour fermer hermétiquement la partie de sortie de médicament liquide (41) ; et
un médicament liquide (100) qui est stocké entre la partie de sortie de médicament liquide (41) et le joint d'étanchéité (43) et peut être déchargé à travers la partie de sortie de médicament liquide (41) par le coulissement du joint d'étanchéité (43).

7. Seringue pré-remplie (1) selon la revendication 6, dans laquelle :
le cylindre de seringue (10) comprend :
un corps de cylindre de seringue (40) comprenant la partie de sortie de médicament liquide (41) à une extrémité distale du corps de cylindre de seringue (40), une ouverture d'insertion (46) ouverte à une extrémité proximale du corps de cylindre de seringue (40), la partie corps (44), et une collerette de cylindre de seringue (45) qui dépasse d'un rebord de l'ouverture d'insertion (46), et où la partie corps (44) est située entre la partie de sortie de médicament liquide (41) et l'ouverture d'insertion (46) et a une forme de tube avec un diamètre extérieur maximum de 12 mm et une longueur totale maximum le long de l'axe central de 60 mm ; et
un tube extérieur (30) comprenant une partie distale de tube (31) ouverte à une extrémité distale du tube extérieur (30) et l'ouverture (39) à une extrémité proximale du tube extérieur (30), où le tube extérieur (30) est formé d'un manchon dans lequel le corps de cylindre de seringue (40) est adapté pour être inséré, où le tube extérieur (30) comprend la partie d'accrochage de doigt (37a) qui est située à au moins 70 mm d'une extrémité proximale de la partie de sortie de médicament liquide (41) et fait saillie depuis une surface circonférentielle extérieure du tube extérieur (30) dans la première direction, où le tube extérieur (30) a une longueur circonférentielle extérieure de 40 à 95 mm au moins près d'une extrémité distale de la partie d'accrochage de doigt (37a), et où le tube extérieur (30) maintient fixement la collerette de cylindre de seringue (45) pour supporter le corps de cylindre de seringue (40) à l'intérieur du tube extérieur (30), et le tube extérieur (30) constitue au moins une partie proximale de la partie de préhension (33).

8. Seringue pré-remplie (1) selon la revendication 7, dans laquelle le tube extérieur (30) s'étend depuis le rebord de l'ouverture d'insertion (46) vers l'extrémité distale du corps de cylindre de seringue (40) de manière à former un manchon entourant une surface latérale de la partie corps (44), la longueur circonférentielle extérieure du tube extérieur (30) allant de 40 à 95 mm.

9. Seringue pré-remplie (1) selon la revendication 8, dans laquelle le tube extérieur (30) comprend un orifice d'inspection (32) qui pénètre dans une paroi du tube extérieur (30) dans une seconde direction perpendiculaire à la première direction dans la direction radiale, la partie corps (44) étant visuellement exposée à travers l'orifice d'inspection (32).

10. Seringue pré-remplie (1) selon la revendication 9, dans laquelle :
le tube extérieur (30) comprend deux paires de nervures (36d) en des positions opposées l'une à l'autre avec l'axe central entre elles, les nervures (36d) de chaque paire dépassant depuis un côté d'une paroi intérieure du tube extérieur (30) et de façon linéaire le long de l'axe central,
chacune des deux paires de nervures (36d) comprend une partie de contact et une partie de support, la partie de contact étant conçue pour venir en contact avec une surface circonférentielle extérieure d'une partie distale du corps de cylindre de seringue (40) de manière à empêcher le corps de cylindre de seringue (40) de s'incliner vers le tube extérieur (30), la partie de support supportant une surface distale de la collerette de cylindre de seringue (45), et
une distance entre les deux paires de nervures (36d) opposées l'une à l'autre est supérieure à une largeur de l'orifice d'inspection (32) le long de la première direction.

11. Seringue pré-remplie (1) selon l'une quelconque des revendications 7 à 10, dans laquelle :
la collerette de cylindre de seringue (45) a un profil extérieur ayant un grand axe et un petit axe perpendiculaire au grand axe, vu depuis un côté proximal de la collerette de cylindre de seringue (45), la collerette de cylindre de seringue (45) comprenant une paire de surfaces côté grand axe prévues aux deux extrémités dans la direction du grand axe et une paire de surfaces côté petit axe prévues aux deux extrémités dans la direction du petit axe, et
le tube extérieur (30) comprend des nervures de restriction de rotation (36c) dépassant chacune de la paroi intérieure du tube extérieur (30) vers la surface côté petit axe de la collerette de cylindre de seringue (45), chaque nervure de restriction de rotation (36c) étant conçue pour venir en contact avec la surface respective côté petit axe pour limiter la rotation du corps de cylindre de seringue (40) par rapport au tube extérieur (30).

12. Seringue pré-remplie (1) selon la revendication 11, dans laquelle le corps de cylindre de seringue (40) comprend un adaptateur luer-lock (42) qui entoure une surface latérale de la partie de sortie de médicament liquide (41) et est adapté pour être fixé en étant vissé à un moyeu (23) avec une aiguille d'injection (22) destinée à administrer le médicament liquide (100).

13. Seringue pré-remplie (1) selon la revendication 11 ou 12, dans laquelle :
le tube extérieur (30) comprend la partie saillante annulaire (37) située entre la partie de préhension (33) et l'ouverture (39), la partie saillante annulaire (37) faisant saillie vers l'extérieur depuis une surface circonférentielle extérieure du tube extérieur (30) de 0,5 à 2 mm ,
la partie d'accrochage de doigt (37a) s'étend dans la première direction de manière à rejoindre une surface circonférentielle extérieure de la partie saillante annulaire (37),
une surface latérale de la partie d'accrochage de doigt (37a) comprend deux sections linéaires parallèles (37a1) qui s'étendent le long de la première direction et une section en arc (37a2) reliant les deux sections linéaires (37a1) en s'étendant le long d'une circonférence extérieure du tube extérieur (30), et
une distance entre les deux sections linéaires (37a1) est approximativement égale à une largeur de la partie saillante annulaire (37) le long d'une seconde direction perpendiculaire à la première direction dans la direction radiale.

14. Seringue pré-remplie (1) selon l'une quelconque des revendications 6 à 13, dans laquelle une distance entre une surface distale de la partie d'accrochage de doigt (37a) et une surface proximale de la partie de pression (53) est de 40 mm au maximum avant l'évacuation du médicament liquide (100) et est adaptée pour être réduite à une valeur de 2 à 10 mm après l'évacuation du médicament liquide (100).
